# NEW EUROPEAN PATENT SPECIFICATION

(11) **EP 0 809 680 B2**
(45) Date of publication and mention of the opposition decision: **07.05.2003**
(45) Mention of the grant of the patent: 02.12.1998
(21) Application number: 96904626.7
(22) Date of filing: 15.02.1996
(51) Int. Cl.: C09J 7/02, C09J 7/04, A61L 15/58, A61L 15/62, A61F 13/15

(54) **USE OF HEAT-SEALABLE FILMS THAT ARE DEGRADABLE FOR DISPOSAL PRODUCTS AND ABSORBENT PRODUCTS CONTAINING THESE FILMS**
VERWENDUNG VON HEISSSIEGELFÄHIGEN, ABBAUBAREN FOLIEN FÜR WEGWERFARTIKEL UND DIESE FOLIEN ENTHALTENDE ABSORBIERENDE ERZEUGNISSE
UTILISATION DE FILMS THERMOSCELLABLES JETABLES ET DEGRADABLES ET PRODUITS ABSORBANTS LES COMPRENANT

(30) Priority: 17.02.1995 US 390465
(43) Date of publication of application: 03.12.1997
(73) Proprietor: McNEIL-PPC, INC., Skillman, New Jersey 08558 (US)
(72) Inventor: LICHSTEIN, Bernard, M., Elizabeth, NJ 07208 (US)
(74) Representative: Groening, Hans Wilhelm, Dipl.-Ing.
(86) International application number: US9601953
(87) International publication number: WO96025468

(56) References cited:
- EP-A- 0 429 708
- EP-A- 0 701 805
- WO-A-92/06173
- WO-A-93/00116
- WO-A-93/04112
- WO-A-94/00293
- DE-A- 4 040 158
- DE-A- 4 308 649
- FR-A- 1 485 578
- GB-A- 65 885
- GB-A- 2 242 630
- GB-A- 2 243 327
- GB-A- 2 265 885
- JP-A- 3 234 800
- US-A- 4 020 228
- US-A- 4 863 450

## Description

### Background of the Invention

This invention relates to the use of a heat sealable degradable sheet in disposable products such as sanitary napkins, tampons, diapers, surgical dressings, wound dressings, wherein a component of the product is a pervious or impervious plastic film; and wherein it is preferred that the plastic film be readily biodegradable, or at the least fragmentable when the product has been discarded after use. More particularly, this invention relates to the use of heat-sealable films that are biodegradable, hydrolyzable or fragmentable, over spans of time compatible with typical transient or treatment times in the waste disposal techniques typically used for the end products and packaging into which such films are incorporated.

Heat-sealable plastic films or sheets have great utility as product packaging, as package outer wraps and as product components. For example, permeable and impermeable components of disposable products such as diapers, sanitary napkins, incontinence products, surgical gowns and hospital underpads may advantageously be made in whole or in part from heat sealable films or sheets.

Conventional films, including heat sealable films, present problems, however, from the standpoint of waste disposal. Few such films are biodegradable, or degradable by other mechanisms in time spans compatible with the transient times that products experience in sewage sludge systems and in commercial composting systems. For example, although future developments may shorten these ranges, anaerobic sewage systems are exposed to and treat a waste load comprised of materials and products within a time span of about thirty (30) days; aerobic sewage systems are exposed to the waste load seven to ten (7-10) days; and current commercial composting systems have waste exposing transit times ranging from several weeks to twenty-four (24) weeks. Landfill exposure times are measured in decades and centuries, although the management of landfills and their possible remediation may shorten the expected exposure times of waste even in this mode of disposal.

With the exception of certain coated papers, among commonly used packaging materials, only uncoated cellophane degrades within the commonly experienced sewage sludge transient times. Cellophane, however, not being thermoplastic, cannot be attached to itself or to other materials except by the use of mechanical folding, crimping, or twisting or by the application of cold glue, hotmelt adhesives, or pressure sensitive adhesives, in areas where adhesion is desired. If one were to apply such adhesives or other coatings over the entire surface of the cellophane (to provide universal adhesion or to modify properties such as water susceptibility and moisture vapor transmission), the biodegradability property of the cellophane would be adversely affected in sewage and composting, and the time needed for degradation in landfills would become greatly and unpredictably lengthened. Similar observations can be made about the slowing of the rates of biodegradation, hydrolysis, fragmentation and flushability of any coated thermoplastic or nonthermoplastic degradable film, such as polycaprolactone, polyparadioxanone, polylactide, polyhydroxybutyrate, polyhydroxyvalerate, their copolymers, modified starches and the like. Once such films are coated, access to them by potential degrading agents such as microorganisms, enzymes, aqueous and other solvolyzing media is blocked or at the very least substantially impeded.

### Summary of the Invention

The present invention provides to the use of a heat sealable degradable sheet in disposable products. In general, it does so by providing a film comprising a degradable film substrate, intermittently coated with a thermoplastic material which will adhere to itself, to the film and to the other substrates and components when the thermoplastic material is heated to its softening point whereby a sharp non-bleeding pattern is created that does not migrate into the regions that should remain uncoated. The coating can be applied to one or both sides of the film. The film thus produced may serve as a product wrap, a packaging wrap, or a perforate, imperforate, or other, plastic component for products such as sanitary napkins, adult incontinence devices, diapers, surgical gowns and hospital underpads. The films comprising the components of such products often must be capable of being thermally fused or sealed to themselves or to other components. In sanitary napkins, adult incontinence devices or surgical and wound dressings, films in accordance with this invention could comprise all or part of the perforate body-facing cover, any intermediate films acting as partial barriers, edge coverings, flaps, tabs and barrier films. In surgical gowns and underpads, they could form the barrier portions of the product. Films in accordance with this invention may also be used for the outer surfaces of a composite cardboard tampon applicator, thus providing a smooth outer surface which facilitates insertion. In packaging such as for tampons, films in accordance with this invention could comprise a wrap for a digital or applicator tampon or for an overwrap for the primary box or package that contains several tampons.

When products made with films in accordance with this invention are exposed to the degrading actions of agents such as microorganisms, fungi, enzymes and hydrolyzing solutions, substantial areas of the degradable film substrate are exposed to these degrading agents. The agents can act directly on, around and beneath the discontinuous thermoplastic coating, even if the coating itself is not degradable. Access to the film substrate is improved with increased discontinuity and exposure between the coated areas, and especially if only one side or large portions of both sides are not coated. It has been found that once large numbers of discontinuities exist, even if their individual areas are small, a typical film substrate's resistance to degradation by virtue of the presence of a nondegradable coating is greatly reduced, to the point of making the film highly susceptible to attack by degrading agents. The fragmented product after degradation is typically a loose residue of thermoplastic fragments whose shape, mass and continuity will be determined by the shape, connections and mass of the original pattern of the coating.

Of course, if the thermoplastic coating is itself degradable, then all of the coated film can degrade and ultimately mineralize, assuming that the mechanism of degradation of the film and coating so dictates.

### Brief Description of Drawings

There are shown in the drawings aspects of certain preferred embodiments, it being understood that the invention is not limited to the precise embodiments and instrumentalities shown.
Figure 1 is a perspective view of a film used in accordance with the invention.
Figure 2 is a partial cross-sectional view, taken along the line 2-2 in Figure 1.
Figure 3 is a top plan view of an exemplary product, a wound dressing, using a heat-sealable film in accordance with the invention.
Figure 4 is a cross-sectional view, taken along the line 4-4 in Figure 3.
Figure 5 is a cross-sectional view of an exemplary product, a sanitary napkin, using heat-sealable films in accordance with the invention.
Figure 6 is a perspective view of an exemplary product, a tampon, using a heat sealable film as a component of the tampon in accordance with the invention.
Figure 7 is a perspective view of an exemplary product, a tampon, using a heat sealable film as an overwrap of the product, in accordance with the invention.
Figure 8 is a perspective view of an exemplary product, a tampon applicator, using a heat sealable film to form a smooth outer surface of the product.

### Detailed Description

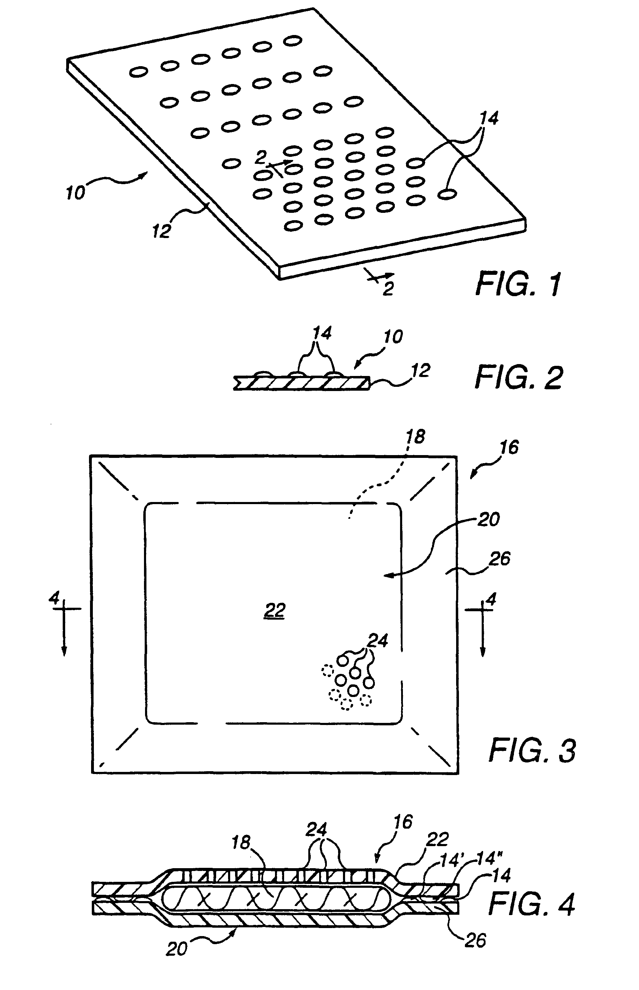

Referring now to the drawings in detail, wherein like reference numerals indicate like elements, there is seen in Figures 1 and 2 a sheet-like film designated by the reference numeral 10. The film 10 includes a substrate 12, with a coating 14 of thermoplastic adhesive disposed on it in a discontinuous pattern.

The substrate 12 may take the form of a film, or, as explained below, a nonwoven fabric. The substrate 12 is degradable by one or more mechanisms, such as biodegradability or hydrolysis.

Examples of films exhibiting degradability, and which may be used as the substrate 12 in accordance with the invention are: cellophane, cellulose acetate, cellulose nitrate, collagen, chitin, chitosan, polycaprolactone, polylactides, polylactic acid, polyglycollates, polyparadioxanone, polyhydroxybutyrate, polyhydroxyvalerate, coplymers of polyhydroxybutyrate and polyhydroxyvalerate, polyvinyl alcohol, polyvinyl acetate, nylons, polyvinylpyrrolidone, carboxyethylcellulose, hydroxyethyl cellulose, polyethylene glycols, methoxypolyethylene glycols, polyethylene oxides and carboxymethylcellulose, hydroxyalkyl celluloses such as hydroxyethylmethyl cellulose, hydroxypropyl cellulose and hydroxypropylethyl cellulose, methylcellulose, plasticized starches and polymer films with high loadings of starch and/or plasticizer where the starch content is greater than 50% by weight. Other suitable materials may occur to those skilled in the art.

Examples of additives which may be incorporated into the materials making up the films used as the substrate 12 are: antiblock agents to reduce unwanted self-adhesion and unwanted adhesion to other surfaces, plasticizers to control strength, extensibility, flexibility and rattle noisiness, antioxidants and other stabilizers to control degradation before and during use, slip agents to reduce friction, antistatic agents, fillers and pigments.

Also suitable materials for use as the substrate 12 are biodegradable nonwoven fabrics made, for example, of cotton, rayon or cotton/rayon blends, various tissues and papers, or other biodegradable fibers and blends of fibers such as wood pulp, flax, jute, ramie, chitin and chitosan. Such nonwovens, when consolidated from a carded or random-laid fibrous web by nonbinder processes, such as hydroentangling, are not in and of themselves heat-sealable. They therefore benefit from the application of discontinuous thermoplastic coatings in accordance with this invention.

Presently preferred as films and nonwovens for the substrate 12 are those substrates which, before degradation, are sufficiently strong and durable to serve their intended purposes, are readily commercially available, and are easy to coat with the thermoplastic coating. It is also preferred, but not necessary, that the substrate be of a composition that degrades within the transient time of the customary and expected disposal means for the product of which it is foreseeably to be a component. For these reasons, cellophane and certain wood-based tissues and papers are examples of preferred substrates. Most preferred, however, is cellophane, for its wide utility and ubiquitousness as a barrier packaging material, its flexibility and its good strength and high film yield characteristics. Other films which may become readily commercially available, and which meet the degradability requirement as determined by the traditional mode of disposal for products into which they are to be incorporated, will also preferred.

The coating 14 may comprise regular and repeating patterns or random arrangements of geometric forms, abstract forms, natural forms and the like. The patterns may be connected or disconnected, so long as a significant number of discontinuities or breaks in the pattern occur to expose uncoated degradable substrate. The coating 14 must be thermoplastic, so that it goes through a tacky phase on being heated, enabling it to seal to itself, to the degradable substrate 12 or to another material or component to which one would want to attach the substrate 12.

Coating processes useful for this invention are those which are capable of yielding the desired discontinuous coating 14 for the substrate 12. The preferred methods of applying the coating 14, described below are primarily adaptations of printing techniques, such as gravure printing and screen printing. Nonimpact techniques, such as fountain and laser jet printing may also be used.

Many coatings 14 useful for this invention are applied as a suspension or solution in a volatile liquid that is subsequently removed or dried or cured in place. Most such formulations are comprised of three components: a binder which is the polymeric material which forms the coating; a pigment when opacity or color is desired; and a volatile liquid carrier which is driven off after the coating is applied. Examples of suitable binders are polyvinyl alcohol, polyvinylidene chloride, polyvinyl chloride, polyvinyl fluoride, polyvinyl acetate, polyacrylates, polyalkylacrylates (such as polymethyl methacrylate), nitrocellulose, ethyl cellulose, cellulose acetate propionate, cellulose acetate butyrate, methyl cellulose, hydroxypropyl cellulose, ethylhydroxyethyl cellulose, ethylene acrylic acid copolymers, ionomer copolymers of ethylene and salts of methacrylic acid and nylons and other polyamides. Others may occur to those skilled in the art. The binder of the coating 14 may be biodegradable or non-biodegradable.

Preferred, however, are coating techniques which deliver dry binder or toners together with the requisite additives and modifiers. Such techniques allow for delivery of a high loading of the thermoplastic component needed for heat sealing, avoidance of dilution of the binder by solvent (and the necessity for removing the solvent and drying or curing the remaining binder), and creation of a sharp non-bleeding pattern that does not migrate into the regions that should remain uncoated.

Examples of techniques that deliver predominantly dry thermoplastic binder are the hotmelt gravure process and hotmelt spray or hotmelt fibrous coating processes. The hotmelt gravure process is capable of creating precise and reproducible geometric or pictorial coating patterns of fine photograph-like detail and precise thicknesses. Those skilled in the art will appreciate that these coating patterns depend on the detail, fineness and depth of the engraved gravure roll or plate and the technique for creating it, e.g. laser or chemical etching. Hotmelt processes are also capable of laying down less well defined coating patterns that can be comprised of any one or a mixture of the following: fine particulates, coarse particulates, short fibers, longer fibers and continuous and discontinuous filaments. The patterns can be random or orderly helicals, swirls and lines.

Additional dry binder coating techniques are those using thermal fusing of electrostatically produced patterns of carbon containing thermoplastics, or xerographically produced patterns of thermoplastic coatings containing photoconductive dyes. The thermoplastic polymer components of these electrostatically transferred toners are epoxy, hydrocarbon, acrylic or polyamide resins. Examples of polymers used in these dry binder techniques are ethylene-vinyl acetate copolymers, polyamides such as the various nylons, polycaprolactone, polyhexamethylene adipamide, styrene isoprene copolymers, styrene butadiene copolymers, polyethylene, polypropylene, polyesters such as polyethylene terephthalate and various polyester and polyether polyurethanes. Other polymers will occur to those skilled in the art.

Examples of additives which may be incorporated into coating formulations are antiblock agents to reduce unwanted self-adherence and unwanted adherence to other surfaces, stabilizers to control degradation during and after coating, slip agents to reduce friction, antistatic agents, fillers and pigments.

Examples of pigments suitable for use in the invention are titanium dioxide and zinc oxide. Extender pigments such as calcium carbonate and silicon dioxide may also be used. Non-white pigments can be used if color is desired.

The utility of the invention was demonstrated by the following Examples:

### 1. Experimental Methods:

a. Aerobic Digestion. Twelve bench scale, stirred aerobic digestion units were set up. The stirring was done by two paddle mixers each with six paddles. Each digestion unit contained a 2000 ml mixture comprised of 1800 ml of a "culture" of freshly collected aerobically digested sludge and 200 ml of a "feed" of return activated sludge (RAS). Digestion units were incubated at 35°C. Each unit was fed 200 ml of 10 g/l RAS every two days until day 10, the end of the trial.

Test samples were rectangular pressed pieces, 0.010 in. thick, weighing about 1 g. Samples were run in duplicate. Each sample was placed in a nylon mesh bag tied to a string paddle and rotated at a moderate speed. Additional air from an aquarium pump was introduced through a diffuser stone to each reactor. pH was monitored. Acid or base was added as necessary to maintain the pH at 7. The following weights were measured: for the samples - starting samples and recovered residues; and for the sludge - total solids and volatile solids (combustible organic solids).

b. Anaerobic Digestion. Sixteen bench scale digestion units, non-stirred, were set up in one incubator at 35°C. Each digestion unit contained a 3650 ml mixture comprised of 2100 ml digested sludge, 1500 ml raw sludge and 50 ml acclimated sludge. Test samples were rectangular pressed pieces, 0.010 in. thick, weighing about 1g. Samples were run in duplicate. Each sample was put in a nylon mesh bag which was inserted into a digestion unit where it was held down with a glass stopper. Two digestion units had similarly held down empty nylon mesh bags as controls. Each unit was connected to a gasometer. Digestion units were manually shaken daily before the volume of gas produced was measured. Each unit was opened on days 10, 20 and 30 to weigh the samples and to measure the following for the sludge - pH, total solids and volatile solids (combustible organic solids).

### 2. Biodegradation Of Uncoated And Coated Cellophanes In Sewage Sludge.

| | Percent Weight Loss | | | |
|---|---|---|---|---|
| | Aerobic | Anaerobic | | |
| | 10 day | 10 day | 20 day | 30 day |
| Cellophane uncoated, 0.0011 in. thick | 80.4 | 100.0 | 100.0 | 100.0 |
| Cellophane 0.0011 in. thick, coated one side with fine dot pattern of ethylene vinyl acetate copolymer, 0.001 in. thick, per this invention | 75.0 | 68.7 | 91.8 | 100.0 |
| Cellophane 0.0011 in. thick, coated each side with fine dot pattern of ethylene vinyl acetate copolymer, 0.001 in. thick, per this invention | 33.5 | 1.2 | 35.8 | 76.8 |
| Cellophane 0.0009 in. thick coated each side with nitrocellulose, 0.00005 in. thick | - | 0.0 | 0.0 | 13.1 |
| Cellophane 0.0007 in. thick coated each side with polyvinylidene chloride, 0.00005 in. thick | 11.8 | 17.4 | 18.2 | 19.2 |

### 3. Sealability:

Both the one side and two side dot pattern EVA coated cellophane was found capable of being sealed to themselves when exposed to a hot plate heat source.

The cellophane films, coated on two sides with nitrocellulose or with polyvinylidene chloride, sealed to themselves when exposed to the heat source.

The uncoated cellophane did not seal to itself when exposed to the heat source.

Referring now to Figure 3 and 4, there is seen a wound dressing made from a film 10 in accordance with the invention. As is apparent from the Figure 4, individual elements 14' and 14" for example, of the coating 14, may be locally heat sealed, as at 16, to form a secure seam. The elements of the coating 14 may be heat sealed to the substrate 22 or to themselves.

Referring now to Figures 3 to 8, exemplary applications of films or sheets 10 in accordance with the invention will now be described.

Figures 3 and 4 illustrate a wound dressing, designated generally by the reference numeral 16. The wound dressing 16 includes an absorbent core 18, enclosed within an envelope 20 of film 22 in accordance with the invention. The film 22 in this particular application may be perforated, as indicated for example at 24, and may advantageously comprise a cellophane substrate and a heat-sealable coating like the coating 14 described above. Thus, referring again to Figures 3 and 4, the margins 26 of the envelope 20 may be heat sealed by fusion of the coating 14 around the periphery of absorbent core 18.

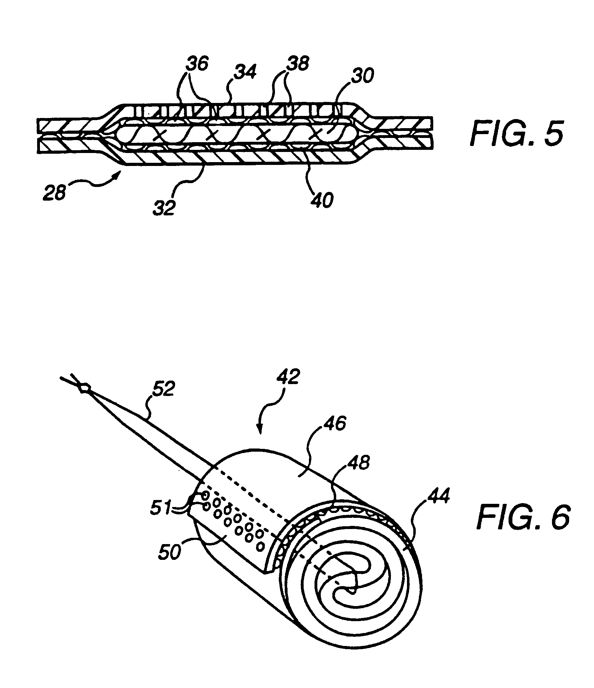

Referring now to Figure 5, there is seen in cross-section a sanitary napkin using films in accordance with the invention. Referring to Figure 5, the sanitary napkin, designated generally by the reference numeral 28, includes an absorbent core 30, a barrier layer 32 and a cover layer 34. The barrier layer 32 may comprise cellophane, coated on one side with a discontinuous coating 40 similar to the above-described coating 14. The cover layer 34 may also be of a cellophane, perforated as indicated at 38, and provided with a discontinuous heat sealable coating 36 like the coating 14. It will be appreciated that the barrier layer 32 and cover layer 34 may thus be also caused to adhere in the manufacture of the sanitary napkin 28 to the absorbent core 30.

Referring now to Figure 6, there is seen a tampon 42 of the kind disclosed in U.S. Patent No. 4,863,450, assigned to the assignee of the present invention. The tampon 42 includes a core 44 of absorbent material, associated with a permeable cover sheet 46, laminated by means of a heat sealable coating 48 to the core 44. The cover sheet 46 may comprise cellophane which may be heat sealed to itself at an overlapping portion 50. The permeable cover sheet 46 may be perforated as indicated at 51 and provided with the above-mentioned coating 48 on one side, or, alternatively, on both sides. A withdrawal string 52 may be attached to the core 44.

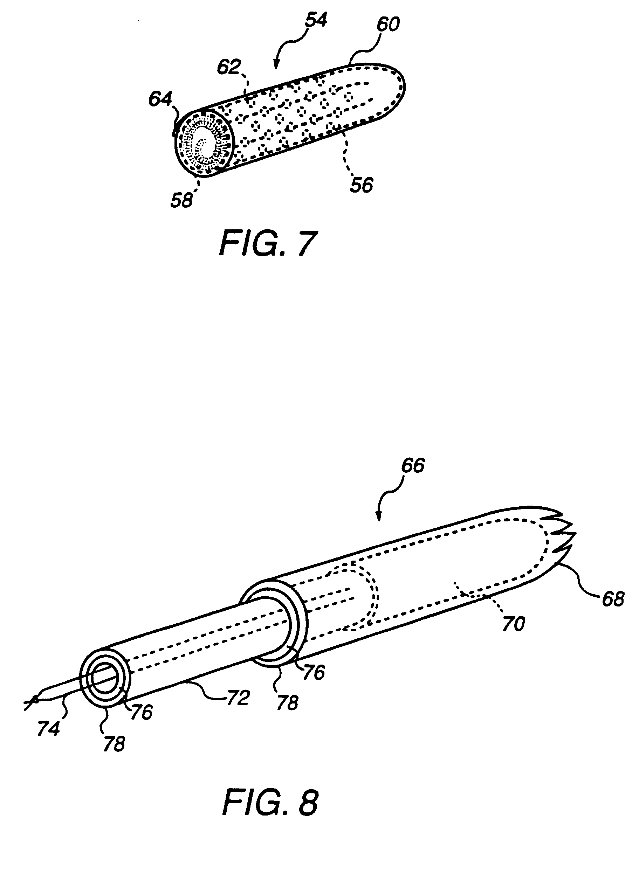

Referring now to Figure 7, there is seen a tampon 54. The tampon 54 includes a generally cylindrical core 56 of absorbent material (shown in phantom) and a withdrawal string 58, (also shown in phantom). The tampon 54 is overwrapped, preferably completely, with an impermeable film 60, provided with a discontinuous heat sealable coating 62, similar to the above-described coating 14, on one side as shown, or, alternatively, on both sides. The overwrap 60 may be heat sealed to itself in the overlapping portion 64.

Referring now to Figure 8, there is seen a tampon applicator, designated generally by the reference numeral 66. The tampon applicator 66 comprises an outer portion 68, which houses a tampon 70. Slidably received in the outer portion 68 is a plunger portion 72. A withdrawal string 74 associated with the tampon 70 extends through the plunger portion 72. The outer portion 68 and plunger portion 72 may be made as a composite, or laminate, of at least two layers: an inner layer 76 of cardboard and an outer layer 78 of heat sealable film in accordance with the invention. The outer layer 78 may be heat sealed to the inner layer 76, heat sealed to itself, or heat sealed to itself and the outer layer.

The present invention may be embodied in other specific forms without departing from its spirit or essential attributes. Accordingly reference should be made to the appended claims, rather than to the foregoing specification, for an indication of the scope of the invention.

## Claims

1. Use of a heat sealable degradable sheet in disposable products which comprises:
a substrate of degradable flexible sheet material; and
a thermoplastic coating intermittently disposed on said substrate, whereby a sharp non-bleeding pattern is created that does not migrate into the regions that should remain uncoated whereby said substrate is accessible to degrading agents notwithstanding the presence of said coating, and said coating being so arranged on said substrate as to render said substrate heat sealable, said substrate being degradable by a mechanism from the group consisting of bio-degradability and hydrolyzability,
wherein the disposable products are selected from the group consisting of sanitary napkins, tampons, diapers, surgical dressings and wound dressings.

2. Use in accordance with claim 1, wherein said substrate comprises a plastic polymeric film.

3. Use in accordance with claim 2, wherein said substrate comprises one of the group consisting of cellophane, cellulose acetate, cellulose nitrate, polycaprolactone, polylactides, polyparadioxanone, polyhydroxybutyrate, polyhydroxyvalerate, copolymers of polyhydroxybutyrate and polyhydroxyvalerate, polyvinyl alcohol, polyvinyl acetate, nylons, polyvinylpyrrolidone, polyethylene oxides and plasticized starches.

4. Use in accordance with claim 2, wherein said coating comprises one of the group consisting of ethylene-vinyl acetate copolymers, polyvinyl alcohol, nylons, polycaprolactone, polyhexamethylene adipamide, styrene isoprene copolymers, styrene butadiene copolymers, polyethylene, polypropylene; polyesters such as polyethylene terephthalate and polyester and polyether polyurethanes.

5. Use in accordance with claim 2, wherein said coating comprises one of the group consisting of polyvinylidene chloride, polyvinyl chloride, polyvinyl fluoride, polyvinyl acetate, polyacrylates, polyalkylacrylates, nitrocellulose, ethyl cellulose, cellulose acetate propionate, cellulose acetate butyrate, methyl cellulose, hydroxypropyl cellulose, ethylhydroxyethyl cellulose, ethylene acrylic acid copolymers, ionomer copolymers of ethylene, salts of methacrylic acid, and nylons.

6. Use in accordance with claim 1, wherein said substrate comprises a biodegradable nonwoven fabric.

7. Use in accordance with claim 6, wherein said substrate is selected from the group consisting of cotton, rayon, cotton/rayon blends, woodpulp, flax, jute, ramie, chitin, chitosan and a combination thereof.

8. Use in accordance with claim 6, wherein said coating comprises one of the group consisting of ethylene-vinyl acetate copolymers, polyvinyl alcohol, nylons, polycaprolactone, polyhexamethylene adipamide, styrene isoprene copolymers, styrene butadiene copolymers, polyethylene, polypropylene, polyesters soch as polyethylene terephthalate and polyester and polyether polyurethanes.

9. Use in accordance with claim 6, wherein said coating comprises one of the group consisting of polyvinylidene chloride, polyvinyl chloride, polyvinyl fluoride, polyvinyl acetate, polyacrylates, polyalkylacrylates, nitrocellulose, ethyl cellulose, cellulose acetate propionate, cellulose acetate butyrate, methyl cellulose, hydroxypropyl cellulose, ethylhydroxyethyl cellulose, ethylene acrylic acid copolymers, ionomer copolymers of ethylene, salts of methacrylic acid, and nylons.

10. An absorbent product comprising an absorbent core and a backing layer, wherein the backing layer comprises a heat sealable degradable sheet comprising a substrate of degradable flexible sheet material and a thermoplastic coating intermittently disposed on said substrate, whereby a sharp non-bleeding pattern is created that does not migrate into the regions that should remain uncoated , whereby said substrate is accessible to degrading agents notwithstanding the presence of said coating, and said coating being so arranged on said substrate as to render said substrate heat sealable, said substrate being degradable by a mechanism from the group consisting of bio-degradability and hydrolyzability, and said sheet being degraded after usage.

11. An absorbent product comprising a top layer, a bottom layer and an absorbent core therebetween, wherein at least one of the layers comprises a heat sealable degradable sheet comprising a substrate of degradable flexible sheet material and a thermoplastic coating intermittently disposed on said substrate, whereby a sharp non-bleeding pattern is created that does not migrate into the regions that should remain uncoated, whereby said substrate is accessible to degrading agents notwithstanding the presence of said coating, and said coating being so arranged on said substrate as to render said substrate heat sealable, said substrate being degradable by a mechanism from the group consisting of bio-degradability and hydrolyzability, and said sheet being degraded after usage.

12. The absorbent product of claim 11 wherein both the top and bottom layers comprise the heat seabble degradable sheet.

13. A tampon comprising a core of absorbent material and a heat sealable degradable permeable cover sheet disposed around and encircling said core, said permeable cover sheet comprising a substrate of degradable flexible sheet material, said permeable cover sheet having intermittently disposed thereon a thermoplastic coating, whereby a sharp non-bleeding pattern is created that does not migrate into the regions that should remain uncoated, whereby said substrate is accessible to degrading agents notwithstanding the presence of said coating, and said coating being so arranged on said substrate as to render said substrate heat sealable, said cover sheet having an overlapping portion thereof heat sealed to itself, said substrate being degradable by a mechanism from the group consisting of: biodegradability and hydrolyzability, and said sheet being degraded after usage.

14. In a degradable tampon applicator, a composite structure comprising an inner layer of cardboard and an outer layer of heat sealable degradable sheet material, said sheet material comprising a substrate of degradable flexible sheet material, a thermoplastic coating intermittently disposed on said substrate, whereby a sharp non-bleeding pattern is created that does not migrate into the regions that should remain uncoated accessible to degrading agents notwith standing the presence of the coating, and said coating being so arranged on said substrate as to render said substrate heat sealable, said substrate being degradable by a mechanism from the group consisting of biodegradability and hydrolyzability, and said sheet being degraded after usage.

15. An article in accordance with claim 14, wherein said substrate is heat sealed to said inner layer.

16. An article in accordance with claim 14, wherein said substrate is heat sealed to itself.

17. An article in accordance with claim 14, wherein said substrate is heat sealed to itself and to said inner layer.

## Patentansprüche

1. Verwendung einer heißsiegelbaren abbaubaren Folie in einem wegwerfbaren Produkt, umfassend
ein Substrat eines abbaubaren flexiblen Folienmaterials; und
eine thermoplastische Beschichtung, die mit Unterbrechungen auf dem Substrat aufgebracht ist, wodurch ein scharfes nicht ausblutendes Muster erzeugt wird, das nicht in Bereiche wandert, die unbeschichtet bleiben sollen, wodurch besagtes Substrat ungeachtet der Anwesenheit der Beschichtung für abbauende Agentien zugänglich ist, und wobei die Beschichtung auf besagtem Substrat derart angeordnet ist, daß dieses heißsiegelbar gemacht wird, wobei das Substrat durch einen Mechanismus abbaubar ist, der aus der Gruppe ausgewählt ist, die biologische Abbaubarkeit und Hydrolysierbarkeit umfaßt, wobei die wegwerfbaren Produkte ausgewählt sind aus der Gruppe bestehend aus Damenbinden, Tampons, Windeln, chirurgischen Verbänden und Wundverbänden.

2. Verwendung nach Anspruch 1, bei der das Substrat einen plastischen Polymerfilm umfaßt.

3. Verwendung nach Anspruch 2, bei der das Substrat eine Substanz aus der Gruppe umfaßt, die aus
Cellophan, Celluloseacetat, Cellulosenitrat, Polycaprolacton, Polylactiden, Polyparadioxanon, Polyhydroxybutyrat, Polyhydroxyvalerat, Copolymeren aus Polyhydroxybutyrat und Polyhydroxyvalerat, Polyvinylalkohol, Polyvinylacetat, Nylon, Polyvinylpyrrolidon, Polyethylenoxiden und plastifizierten Stärken besteht.

4. Verwendung nach Anspruch 2, bei der die Beschichtung eine Substanz aus der Gruppe umfaßt, die aus
Ethylen-Vinylacetat-Copolymeren, Polyvinylalkohol, Nylon, Polycaprolacton, Polyhexamethylenadipinsäurediamid, Styrol-Isopren-Copolymeren, Styrol-Butadien-Copolymeren, Polyethylen, Polypropylen, Polyestern wie Polyethylenterephthalat und Polyester- und Polyetherpolyurethanen besteht.

5. Verwendung nach Anspruch 2, bei der die Beschichtung eine Substanz aus der Gruppe umfaßt, die aus
Polyvinylidenchlorid, Polyvinylchlorid, Polyvinylfluorid, Polyvinylacetat, Polyacrylaten, Polyalkylacrylaten, Nitrocellulose, Ethylcellulose, Celluloseacetatpropionat, Celluloseacetatbutyrat, Methylcellulose, Hydroxypropylcellulose, Ethylhydroxyethylcellulose, Ethylenacrylsäurecopolymeren, Ionomercopolymeren des Ethylens, Salzen der Methacrylsäure und Nylon besteht.

6. Verwendung nach Anspruch 1, bei der das Substrat ein biologisch abbaubares Vliesgewebe umfaßt.

7. Verwendung nach Anspruch 6, bei der das Substrat aus der Gruppe ausgewählt ist, die aus
Baumwolle, Reyon, Baumwolle-/Reyonmischungen, Holzzellstoff, Flachs, Jute, Ramiefaser, Chitin, Chitosan und einer Mischung davon besteht.

8. Verwendung nach Anspruch 6, bei der die Beschichtung eine Substanz aus der Gruppe umfaßt, die aus
Ethylen-Vinylacetat-Copolymeren, Vinylalkohol, Nylon, Polycaprolacton, Polyhexamethylenadipinsäurediamid, Styrol-Isopren-Copolymeren, Styrol-Butadien-Copolymeren, Polyethylen, Polypropylen, Polyestern wie Polyethylenterephthalat und Polyester- und Polyetherpolyurethanen besteht.

9. Verwendung nach Anspruch 6, bei der die Beschichtung eine Substanz aus der Gruppe umfaßt, die aus
Polyvinylidenchlorid, Polyvinylchlorid, Polyvinylfluorid, Polyvinylacetat, Polyacrylaten, Polyalkylacrylaten, Nitrocellulose, Ethylcellulose, Celluloseacetatpropionat, Celluloseacetatbutyrat, Methylcellulose, Hydroxypropylcellulose, Ethylhydroxyethylcellulose, Ethylen-Acrylsäure-Copolymeren, Ionomercopolymeren des Ethylens, Salzen der Methacrylsäure und Nylon besteht.

10. Absorbierendes Erzeugnis, das einen absorbierenden Kern und eine Trägerschicht umfaßt, bei dem die Trägerschicht eine heißsiegelbare abbaubare Folie umfaßt, die ein Substrat aus einem abbaubaren flexiblen Folienmaterial und eine thermoplastische Beschichtung, die auf dem Substrat mit Unterbrechungen aufgebracht ist, umfaßt, wodurch ein scharfes nicht ausblutendes Muster erzeugt wird, das nicht in Bereiche wandert, die unbeschichtet bleiben sollen, wodurch das Substrat ungeachtet der Anwesenheit der Beschichtung für abbauende Agentien zugänglich ist, und die Beschichtung auf dem Substrat derart angeordnet ist, daß das Substrat heißsiegelbar wird, wobei das Substrat durch einen Mechanismus abbaubar ist, der aus der Gruppe ausgewählt ist, die biologische Abbaubarkeit und Hydrolysierbarkeit umfaßt, und wobei besagte Folie nach dem Gebrauch abgebaut wird.

11. Absorbierendes Erzeugnis, das eine Deckschicht, eine Unterschicht und einen absorbierenden Kern dazwischen umfaßt, bei dem mindestens eine der Schichten eine heißsiegelbare abbaubare Folie umfaßt, die ein Substrat aus abbaubarem flexiblen Folienmaterial und eine thermoplastische Beschichtung, die auf dem Substrat mit Unterbrechungen aufgebracht ist, umfaßt, wodurch ein scharfes nicht ausblutendes Muster erzeugt wird, das nicht in Bereiche wandert, die unbeschichtet bleiben sollen, wodurch das Substrat ungeachtet der Anwesenheit der Beschichtung für abbauende Agentien zugänglich ist, und die Beschichtung auf dem Substrat derart angeordnet ist, daß das Substrat heißsiegelbar wird, wobei das Substrat durch einen Mechanismus abbaubar ist, der aus der Gruppe ausgewählt ist, die biologische Abbaubarkeit und Hydrolysierbarkeit umfaßt, und wobei besagte Folie nach dem Gebrauch abgebaut wird.

12. Absorbierendes Erzeugnis nach Anspruch 11, bei dem sowohl Deck- als auch Unterschicht die heißsiegelbare, abbaubare Folie umfassen.

13. Tampon, der einen Kern aus absorbierendem Werkstoff und eine heißsiegelbare, abbaubare durchlässige Deckfolie, die um den Kern herum angebracht ist und ihn einschließt, umfaßt, wobei die durchlässige Deckfolie ein Substrat aus abbaubarem flexiblen Folienmaterial umfaßt, wobei die durchlässige Deckfolie darauf mit Unterbrechungen eine thermoplastische Beschichtung aufgebracht hat, wodurch ein scharfes nicht ausblutendes Muster erzeugt wird, das nicht in Bereiche wandert, die unbeschichtet bleiben sollen, wodurch das Substrat ungeachtet der Anwesenheit der Beschichtung für abbauende Agentien zugänglich ist, und die Beschichtung auf dem Substrat derart angeordnet ist, daß das Substrat heißsiegelbar wird, und wobei die Deckfolie mit einem überlappenden Teil derselben an sich selbst heißgesiegelt ist, wobei das Substrat durch einen Mechanismus abbaubar ist, der aus der Gruppe ausgewählt ist, die biologische Abbaubarkeit und Hydrolysierbarkeit umfaßt, und wobei besagte Folie nach dem Gebrauch abgebaut wird.

14. Verbundstruktur in einem abbaubaren Applikationsgerät für Tampons, die eine innere Schicht aus Pappe und eine äußere Schicht aus heißsiegelbarem, abbaubarem Folienmaterial umfaßt, wobei das Folienmaterial ein Substrat aus abbaubarem flexiblen Folienmaterial umfaßt und eine thermoplastische Beschichtung mit Unterbrechungen auf dem Substrat aufgebracht ist, wodurch ein scharfes nicht ausblutendes Muster erzeugt wird, das nicht in Bereiche wandert, die unbeschichtet bleiben sollen, und welches ungeachtet der Anwesenheit der Beschichtung für abbauende Agentien zugänglich ist und wobei die Beschichtung derart auf dem Substrat angeordnet ist, daß das Substrat heißsiegelbar wird, wobei das Substrat durch einen Mechanismus abbaubar ist, der aus der Gruppe ausgewählt ist, die biologische Abbaubarkeit und Hydrolysierbarkeit umfaßt, und wobei besagte Folie nach dem Gebrauch abgebaut wird.

15. Erzeugnis nach Anspruch 14, bei dem das Substrat an die innere Schicht heißgesiegelt ist.

16. Erzeugnis nach Anspruch 14, bei dem das Substrat an sich selbst heißgesiegelt ist.

17. Erzeugnis nach Anspruch 14, bei dem das Substrat an sich selbst und an die innere Schicht heißgesiegelt ist.

## Revendications

1. Utilisation d'une feuille dégradable thermoscellable dans les produits d'emballage jetables, comprenant :
* un substrat de matière souple dégradable ; et
* un revêtement thermoplastique disposé de façon intermittente sur ledit substrat grâce à quoi un motif sans bavure et net est créé, qui dans les régions qui doivent demeurer non revêtues de sorte que ledit substrat est accessible aux agents de dégradation malgré la présence dudit revêtement et ledit revêtement étant arrangé sur ledit substrat de façon à rendre ledit substrat thermoscellable, ledit substrat étant dégradable par un mécanisme choisi parmi la biodégradabilité et l'hydrolysabilité.
dans laquelle les produits jetables sont sélectionnés à partir du groupe constitué des serviettes hygiéniques, des tampons, des couches, des pansements chirurgicaux et des pansements pour les plaies.

2. Utilisation selon la revendication 1, dans laquelle ledit substrat comprend un film polymère plastique.

3. Utilisation selon la revendication 2, dans laquelle ledit substrat comprend un élément du groupe consistant en le cellophane, l'acétate de cellulose, le nitrate de cellulose, la polycaprolactone, les polyactides, la polyparadioxanone, le polyhydroxybutyrate, le polyhydroxyvalérate, les copolymères de polyhydroxybutyrate et de polyhydroxyvalérate, l'alcool polyvinylique, le polyacétate de vinyle, les Nylons, la polyvinylpyrrolidone, les polyoxydes d'éthylène et les amidons plastifiés.

4. Utilisation selon la revendication 2, dans laquelle ledit revêtement comprend un élément du groupe consistant en les copolymères éthylène/acétate de vinyle, l'alcool polyvinylique, les Nylons, la polycaprolactone, le polyhexaméthylène - adipamide, les copolymères styrène/isoprène, les copolymères styrène/butadième, le polyéthylène, le polypropylène, les polyesters tels que le téréphtalate de polyéthylène et les polyuréthannes de polyester et polyéther.

5. Feuille selon la revendication 2, dans laquelle ledit revêtement comprend un élément du groupe consistant en le polychlorure de vinylidène, le polychlorure de vinyle, le polyfluorure de vinyle, le polyacétate de vinyle, les polyacrylates, les polyacrylates d'alkyle, la nitrocellulose, l'éthylcellulose, le propionate d'acétate de cellulose, le butyrate d'acétate de cellulose, la méthylcellulose, l'hydroxypropyle cellulose, l'éthylhydroxyéthylcellulose, les copolymères éthylène/acide acrylique, les copolymères ionomères d'éthylène, les sels d'acide méthacrylique et les Nylons.

6. Utilisation selon la revendication 1, dans laquelle ledit substrat comprend un textile non tissé biodégradable.

7. Utilisation selon la revendication 6, dans laquelle ledit substrat est choisi parmi les coton, rayonne, mélanges de coton/rayonne, pâte à papier, lin, jute, ramie, chitine, chitosan et leurs combinaisons.

8. Utilisation selon la revendication 6, dans laquelle ledit revêtement comprend un élément consistant en les copolymères éthylène/acétate de vinyle, l'alcool polyvinylique, les Nylons, la polycaprolactone, le polyhexaméthylène - adipamide, les copolymères styrène/isoprène, les copolymères styrène/butadiène, le polyéthylène, le polypropylène, les polyesters tels que le téréphtalate de polyéthylène et les polyuréthanes de polyester et polyéther.

9. Utilisation selon la revendication 6, dans laquelle ledit revêtement comprend un élément du groupe consistant en le polychlorure de vinylidène, le polychlorure de vinyle, le polyfluorure de vinyle, le polyacétate de vinyle, les polyacrylates, les polyalkylacrylates, la nitrocellulose, l'éthylcellulose, le propionate d'acétate de cellulose, le butyrate d'acétate de cellulose, la méthylcellulose, la méthylcellulose, l'hydroxypropylcellulose, l'éthylhydroxyéthylcellulose, les copolymères éthylène/acide acrylique, les copolymères ionomères d'éthylène, les sels d'acide méthacrylique et les Nylons.

10. Produit absorbant comprenant un noyau absorbant et une couche de renforcement, dans lequel la couche de renforcement comprend une feuille dégradable thermoscellable comprenant un substrat de matière en feuille souple dégradable et un revêtement thermoplastique disposé de façon intermittente sur ledit substrat grâce à quoi un motif sans bavure et net est créé qui ne migre pas dans les régions qui doivent demeurer non revêtues, de telle sorte que ledit substrat est accessible aux agents de dégradation malgré la présence dudit revêtement et ledit revêtement étant arrangé sur ledit substrat de façon à rendre ledit substrat thermoscellable, ledit substrat étant dégradable par un mécanisme choisi parmi la biodégradabilité et l'hydrolysabilité.

11. Produit absorbant comprenant une couche supérieure, une couche inférieure et entre elles un noyau absorbant, dans lequel au moins l'une des couches comprend une feuille thermoscellable comprenant un substrat de matière en feuille souple dégradable et un revêtement thermoplastique disposé de façon intermittente sur ledit substrat grâce à quoi un motif sans bavure et net est créé qui ne migre pas dans les régions qui doivent demeurer non revêtues, de sorte que ledit substrat est accessible aux agents de dégradation malgré la présence dudit revêtement et ledit revêtement étant arrangé sur ledit substrat de façon à rendre ledit substrat thermoscellable, ledit substrat étant dégradable par un mécanisme choisi parmi la biodégradabilité et l'hydrolysabilité, et ladite feuille étant dégradée après utilisation.

12. Produit absorbant selon la revendication 11, dans lequel les deux couches supérieure et inférieure comprennent la feuille dégradable thermoscellable.

13. Tampon comprenant un noyau de matière absorbante et une feuille de couverture perméable dégradable thermoscellable disposée autour et encerclant ledit noyau, ladite feuille de couverture perméable comprenant un substrat de matière souple dégradable, ladite feuille de couverture perméable ayant disposée sur elle un revêtement thermoplastique intermittent grâce à quoi un motif sans bavure et net est créé qui ne migre pas dans les régions qui doivent demeurer non revêtues, de sorte que ledit substrat est accessible aux agents de dégradation malgré la présence dudit revêtement et ledit revêtement étant arrangé sur ledit substrat de façon à rendre ledit substrat thermoscellable, ladite feuille de couverture ayant une portion en chevauchement qui est thermoscellée sur elle, ledit substrat étant dégradable par un mécanisme choisi parmi la biodégradabilité et l'hydrolysabilité et ladite feuille étant dégradée après utilisation.

14. Structure composite, dans un applicateur de tampon dégradable, comprenant une couche interne de carton et une couche externe d'une matière en feuille dégradable thermoscellable, ladite matière en feuille comprenant un substrat de matière en feuille souple dégradable, un revêtement thermoplastique disposé de façon intermittente sur ledit substrat grâce à quoi un motif sans bavure et net est créé qui ne migre pas dans les régions qui doivent demeurer non revêtues, et est accessible aux agents de dégradation malgré la présence dudit revêtement et ledit revêtement étant arrangé sur ledit substrat de façon à rendre ledit substrat thermoscellable, ledit substrat étant dégradable par un mécanisme choisi parmi la biodégradabilité et l'hydrolysabilité, et ladite feuille étant dégradée après utilisation.

15. Article selon la revendication 14, dans lequel ledit substrat est thermoscellé sur ladite couche interne.

16. Article selon la revendication 14, dans lequel ledit substrat est thermoscellé sur lui-même.

17. Article selon la revendication 14, dans lequel ledit substrat est thermoscellé sur lui-même et sur laite couche interne.
